# EUROPÄISCHE PATENTSCHRIFT

(11) **EP 3 334 366 B1**
(45) Veröffentlichungstag und Bekanntmachung des Hinweises auf die Patenterteilung: **17.11.2021**
(21) Anmeldenummer: 16747466.7
(22) Anmeldetag: 25.07.2016
(51) Int. Cl.: A61B 90/30, A61B 90/00, F21V 23/04, F21V 21/40

(54) **HANDGRIFFVORRICHTUNG FÜR EINE OPERATIONSLEUCHTE MIT SPRACHSTEUERUNG SOWIE OPERATIONSLEUCHTE**
HANDLE DEVICE FOR A SURGICAL LIGHT WITH VOICE CONTROL, AND SURGICAL LIGHT
DISPOSITIF DE POIGNÉE POUR SCIALYTIQUE À COMMANDE VOCALE ET SCIALYTIQUE

(30) Priorität: 13.08.2015 DE 102015113338
(43) Veröffentlichungstag der Anmeldung: 20.06.2018
(73) Patentinhaber: Karl Leibinger Medizintechnik GmbH & Co. KG, 78570 Mühlheim (DE)
(72) Erfinder: STRÖLIN, Joachim, 78604 Rietheim (DE)
(74) Vertreter: Winter, Brandl - Partnerschaft mbB
(86) Internationale Anmeldenummer: PCT/EP2016/067691
(87) Internationale Veröffentlichungsnummer: WO 2017/025309

(56) Entgegenhaltungen:
- EP-A1- 2 590 427
- EP-A2- 0 933 973
- WO-A1-2013/173258
- WO-A1-2015/010757
- CN-U- 204 231 500
- US-A- 5 803 905

## Beschreibung

Die Erfindung betrifft eine Handgriffvorrichtung für eine Operationsleuchte, mit einem, zum Anbringen an einem Leuchtenaufnahmekörper der Operationsleuchte vorbereiteten Griffelement (auch als Handgriff oder schlicht als Griff bezeichnet), welches Griffelement an einem Außenbereich eine Grifffläche ausbildet. Die Erfindung betrifft auch eine Operationsleuchte mit einer solchen Handgriffvorrichtung.

Aus dem Stand der Technik ist es bisher allgemein bekannt, Bedienungseinrichtungen vorzusehen, die in der Operationsleuchte angebracht sind. Mit diesen Bedienungseinrichtungen können auch Sprachsteuerungen verbunden sein, wodurch die Bedienung der Operationsleuchte mittels Spracheingabe ermöglicht wird.

Weiterer Stand der Technik ist aus der EP 2 590 427 A1, der WO 2013/173258 A1, der US 5 803 905 A, der EP 0 933 973 A2, der WO 2015/010757 A1 und der CN 204 231 500 U bekannt.

Dabei hat es sich jedoch als nachteilig herausgestellt, dass die Bedienungseinrichtungen häufig relativ komplex aufgebaut und folglich auch relativ aufwendig in ihrer Herstellung sind. Zudem sind mit der Sprachsteuerung zumeist relativ unhandliche Mikrofone, beispielsweise in Form von Headsets umgesetzt, die schwierig am steril verkleideten Operateur befestigbar sind oder den Operateur gar bei der Operation behindern können.

Es ist somit die Aufgabe der vorliegenden Erfindung, diese aus dem Stand der Technik bekannten Nachteile zu beheben und eine Sprachsteuerung für eine Operationsleuchte zu ermöglichen, die zum einen eine kostengünstige Herstellung der Operationsleuchte ermöglicht, zum anderen den Operateur bei seinen Operationstätigkeiten möglichst wenig behindern soll.

Dies wird erfindungsgemäß durch den kennzeichnenden Teil des Anspruchs 1 gelöst, wobei eine Handgriffvorrichtung vorgesehen ist, mit deren Griffelement eine zumindest einen Schallsensor aufweisende Sprachsteuerungsmodul wieder abnehmbar verbunden ist. Das Sprachsteuerungsmodul weist weiterhin eine Rechnereinheit auf, welche Rechnereinheit (auch als Recheneinheit bezeichnet) elektrisch mit dem zumindest einen Schallsensor verbunden ist und zum Erzeugen von Steuersignalen zum Steuern der Operationsleuchte in Abhängigkeit von den mittels dem zumindest einen Schallsensor ermittelten Messdaten ausgebildet ist.

Durch die Ausgestaltung eines solchen Sprachsteuerungsmoduls ist es gewährleistet, dass der Operateur bei der Operation stets unbehindert durch reine Sprachbefehle /eingaben sowohl die Operationsleuchte selbst, als auch verschiedene, mit der Operationsleuchte gekoppelte Einrichtungen, bspw. Kameraaufnahmen berührungslos bedienen kann. Durch die Anordnung des Sprachsteuerungsmoduls in der Handgriffvorrichtung ist es zudem selbständig gewährleistet, dass der Operateur während der Operation in Reichweite des Schallsensors ist, da er ohnehin zumeist in der Nähe der Operationsleuchte steht. Dadurch ist auch gewährleistet, dass die Spracheingaben des Operateurs auch mit einer relativ hohen Sicherheit durch das Sprachsteuerungsmodul richtig erkannt werden können. Im Weiteren ist es durch den modularen Aufbau möglich, wenn der Endverbraucher einer bisher ohne Sprachsteuerungsmodul ausgebildeten Operationsleuchte auf eine Operationsleuchte mit Sprachsteuerungsmodul wechseln möchte, einfach die bestehende Handgriffvorrichtung durch die Handgriffvorrichtung der erfindungsgemäßen Art auszutauschen. Es muss daher keine komplett neue Operationsleuchte angeschafft werden, wodurch der Einsatzbereich der Handgriffvorrichtung weiter verbessert wird. Da die bekannten Handgriffvorrichtungen ohnehin zumeist austauschbar sind, um sie zur Sterilisation abzunehmen, ist dadurch auch der Aufwand zum Nachrüsten der Operationsleuchte besonders gering. Zudem ist dadurch eine besonders direkte elektrische Anbindung / Versorgung des zumindest einen Schallsensors umgesetzt. Im Betrieb kann die Operationsleuchte dann in Abhängigkeit dieser Steuersignale bspw. unmittelbar derart angesteuert werden, dass Leuchtelemente / Einzelleuchten der Operationsleuchte herunter gedimmt oder ausgeschaltet werden können.

Weitere vorteilhafte Ausführungsformen sind in den Unteransprüchen beansprucht und nachfolgend näher erläutert.

Weiterhin vorteilhaft ist es, wenn das Sprachsteuerungsmodul ein Gehäuse aufweist, in dem der zumindest eine Schallsensor aufgenommen / angebracht / befestigt ist. Als Schallsensor kann ein Mikrofon eingesetzt werden. Dadurch ist eine möglichst robuste Ausführung des Sprachsteuerungsmoduls ausgebildet und der Schallsensor vor der Umgebung weitestgehend geschützt.

Im Weiteren ist es auch vorteilhaft, wenn mehre Schallsensoren in dem Sprachsteuerungsmodul vorhanden sind, die vorzugsweise in unterschiedliche Raumrichtungen ausgerichtet sind. Dadurch ist eine noch sensiblere Spracheingabe möglich.

In diesem Zusammenhang ist es auch weiterhin von Vorteil, wenn das Gehäuse einen scheibenförmigen Hohlkörperabschnitt aufweist / ausbildet, innerhalb dessen der zumindest eine Schallsensor angeordnet / befestigt ist. Dadurch ist das Sprachsteuerungsmodul besonders kompakt ausgestaltet und nimmt möglichst wenig Bauraum ein.

Ist das Gehäuse im Bereich des zumindest einen Schallsensors durchlässig für ein durch den zumindest einen Abstandssensor zu detektierendes Schallsignal (d.h. schalldurchlässig) ausgebildet, ist der zumindest eine Schallsensor besonders vorteilhaft, geschützt von der Umgebung, in dem Gehäuse aufgenommen.

Zweckmäßig ist es auch, wenn das Sprachsteuerungsmodul einen Handgriffaufnahmeabschnitt aufweist, der wiederabnehmbar mit dem Griffelement verbunden ist. Dadurch ist ein zuvor unsteril gemachtes Griffelement durch ein neues / steriles Griffelement besonders einfach auszutauschen, ohne dabei das Sprachsteuerungsmodul selbst mit abnehmen zu müssen.

In diesem Zusammenhang ist es auch von Vorteil, wenn der Handgriffaufnahmeabschnitt einen Aufnahmezapfen ausbildet, auf dem in zumindest einem Betriebszustand der Handgriffvorrichtung ein, die Grifffläche aufweisender Hülsenabschnitt des Griffelementes aufgesteckt / aufgeschoben, d.h. formschlüssig aufgebracht ist. Dadurch ist eine besonders stabile Halterung des Griffelementes relativ zu dem Sprachsteuerungsmodul umgesetzt.

In diesem Zusammenhang ist es auch vorteilhaft, wenn das Griffelement form- und/oder kraftschlüssig mit dem Sprachsteuerungsmodul in zumindest einem Betriebszustand verbunden ist, da dadurch der Austausch des Griffelementes besonders rasch erfolgen kann.

Die Rechnereinheit des Sprachsteuerungsmodul ist weiter bevorzugt derart mit einer zentralen Steuerungseinheit der Operationsleuchte im Betrieb verbunden, dass die Einzelleuchten der Operationsleuchte individuell bedienbar / betätigbar sind. Auch können mittels des Sprachsteuerungsmoduls noch zahlreiche weitere Funktionen umgesetzt sein und etwa Steuersignale erzeugt werden, die die Höheneinstellung oder die Neigung der Operationsleuchte verändern. Dadurch ist es möglich, im Betrieb einen direkten Kontakt des Operateurs mit der Operationsleuchte zu vermeiden.

Vorteilhaft ist es auch, wenn das Sprachsteuerungsmodul (vorzugsweise die Rechnereinheit) mit einer Bildaufzeichnungseinrichtung (elektrisch / kabelgebunden oder drahtlos) verbunden ist. Dadurch ist auch eine Bildaufzeichnungseinrichtung besonders einfach bedienbar. Weiter bevorzugt ist diese Bildaufzeichnungseinrichtung ebenfalls in der Handgriffvorrichtung (vorzugsweise in einem eigenen Modul oder in dem Sprachsteuerungsmodul) integriert. Damit wir insbesondere Bauraum deutlich eingespart.

Vorteilhaft ist es im Weiteren, wenn in dem Sprachsteuerungsmodul zumindest ein Abstandssensor enthalten ist, der zum Detektieren einer Position eines Objektes ausgelegt ist. Dadurch ist es möglich, neben der Bedienung über Spracheingabe, auch gleichzeitig eine Helligkeitsregelung mittels Abstandssensoren umzusetzen.

Ist zudem der zumindest eine Abstandssensor als ein Infrarotsensor ausgebildet, ist das Sprachsteuerungsmodul mit bewährten Messsensoren ausgestattet, die eine noch kostengünstigere Herstellung der Handgriffvorrichtung ermöglichen.

In diesem Zusammenhang ist es auch zweckmäßig, den zumindest einen Abstandssensor elektronisch mit der Rechnereinheit zu verbinden. Dadurch wird der Aufbau des Sprachsteuerungsmoduls weiter vereinfacht.

Im Weiteren umfasst die Erfindung eine Operationsleuchte mit einer Handgriffvorrichtung nach zumindest einer der zuvor genannten Ausführungsformen, wobei die Handgriffvorrichtung zumindest teilweise wieder abnehmbar mit einem Leuchtenaufnahmekörper der Operationsleuchte verbunden ist, wodurch die Sterilisierbarkeit der Handgriffvorrichtung besonders einfach umgesetzt ist.

In diesem Zusammenhang ist es auch von Vorteil, wenn das Sprachsteuerungsmodul wieder abnehmbar an dem Leuchtenaufnahmekörper angebracht ist. Dann kann das Sprachsteuerungsmodul bei Bedarf rasch von der Operationsleuchte nach einem vorhergehenden Befestigen, wieder entfernt werden. Dadurch ist die Operationsleuchte besonders individuell ausrüstbar.

Ist eine Recheneinheit des Sprachsteuerungsmoduls draht- / kabelgebunden / elektrisch mit einer zentralen Steuereinheit der Operationsleuchte verbunden, können Steuersignale von dem Sprachsteuerungsmodul direkt auf die Operationsleuchte übertragen werden, wodurch die Operationsleuchte ihre Einzelleuchten (insbesondere hinsichtlich ihrer Helligkeit / Beleuchtungsstärke) individuell und direkt einstellen kann. Die Ansteuerung der Operationsleuchte ist somit möglichst direkt umgesetzt.

Zweckmäßig ist es stattdessen auch, wenn die Rechnereinheit des Sprachsteuerungsmoduls mittels einer drahtlosen Datenverbindung (vorzugsweise über eine "Bluetooth"-Datenverbindung) mit einer zentralen Steuereinheit der Operationsleuchte verbunden ist. Dadurch ist der Aufbau besonders einfach gehalten.

Erfindungsgemäß ist somit eine Handgriffvorrichtung für eine Operationsleuchte umgesetzt, die über Sprache gesteuert werden kann, ohne dabei berührt werden zu müssen. Der Operateur braucht die Operationsleuchte daher nicht anzufassen, wodurch keine Gefahr besteht, dass er sich während der Operation unsteril macht. Der Vorteil, wenn die Sprachsteuerung (das Sprachsteuerungsmodul) oder Teile davon in der zentralen Handgriffgruppe / Handgriffvorrichtung untergebracht ist / sind, ist, dass es für diese Funktion der ideale Platz dafür ist, da der Anwender zur Befehlsgabe am nächsten ist. Der Operateur steht fast immer genau unter der Operationsleuchte und ist deshalb nur wenige cm oder max. 0,5 m von dem zentralen Handgriff / der Handgriffvorrichtung entfernt.

Die Sprachsteuerungen des Standes der Technik sind oft teuer, wodurch, wenn sie und im Leuchtenaufnahmekörper fest eingebaut sind, ein hoher Anschaffungspreis resultiert. Die Sprachsteuerung oder Teile davon befinden sich erfindungsgemäß in der zentralen Handgriffeinheit der Operationsleuchte. Diese zentrale Handgriffeinheit ist leicht austauschbar. So kann eine Operationsleuchte sehr einfach und schnell mit der Sprachsteuerung nachgerüstet werden. In einer ersten Variante gibt es unterschiedliche Handgriffgruppen, eine mit und eine ohne Sprachsteuerung, wobei diese dann einfach zu tauschen sind. In einer zweiten Variante ist die Sprachsteuerung als Zwischenring zwischen Handgriffgruppe und Leuchtenaufnahmekörper eingebaut. Bei Anordnung des Sprachmoduls / Sprachsteuerungsmoduls in / an dem zentralen Handgriff / der zentralen Handgriffvorrichtung, sind auch Mikrofon / Schallsensor und Lautsprecher des Sprachmoduls im zentralen Handgriff angeordnet. Die Sprachsteuerung / das Sprachsteuerungsmodul könnte alternativ auch direkt mit einer eingebauten Kamera einer Bildaufzeichnungseinrichtung kommunizieren ohne den Umweg über die Operationsleuchtenelektronik (d.h. die zentrale Steuereinheit) zu gehen. Denkbar wäre auch eine Video-Aufnahmefunktion der Kamera z.B. auf eine Speicherkarte direkt in der Handgriffgruppe. Die Aufnahme wird dabei vorzugsweise über die Sprachsteuerung gestartet / gestoppt. Denkbar wäre auch eine Sprachmemo-Aufnahme über die Sprachsteuerung direkt auf die integrierte Speicherkarte.

Denkbar ist ferner, dass die Sprachsteuerung einer Freisprecheinrichtung entspricht und darüber auch Telefongespräche geführt werden können. Das Sprachmodul stellt mit einem beliebigen Mobiltelefon / Handy (Operateur) eine Verbindung her (z.B. über Bluetooth). So kann der Anwender auf seine Kontakte zugreifen und ein Telefongespräch führen und gleichzeitig oder zeitlich versetzt mit der Sprachsteuerung die OP-Leuchte steuern.

In dem Zusammenhang ist es sinnvoll eine Schallausgabeeinheit, wie einen Lautsprecher, einzusetzen. Wegen des Lautsprechereinsatzes kann herkömmliche Musik eingespielt werden. Dabei ist es von Vorteil, wenn der Lautsprecher direkt hinter einer abwischbaren Membran positioniert ist.

Die Steuerung der OP-Leuchte kann zusätzlich oder alternativ zum Abfassen von Nachrichten, Durchführen von Telefonaten, Steuern von Videokameras und/oder Fotokameras bzw. dem Einspielen von Musik genutzt werden.

Die Erfindung wird nun nachfolgend anhand von Figuren näher erläutert, in welchem Zusammenhang auch verschiedene Ausführungsbeispiele beschrieben sind.

Es zeigen:
- Fig. 1: eine isometrische Darstellung einer erfindungsgemäßen Handgriffvorrichtung nach einem ersten Ausführungsbeispiel, in der ein Griffelement fest mit einem Sprachsteuerungsmodul verbunden ist,
- Fig. 2: eine isometrische Darstellung der Handgriffvorrichtung aus Fig. 1, wobei nun lediglich das Sprachsteuerungsmodul dargestellt ist und das Griffelement von einem Aufnahmezapfen des Sprachsteuerungsmoduls abgenommen ist, und wobei ein einen Schallsensor umschließendes Elektronikgehäuse hinter der leicht transparent dargestellten Außenwandung des Gehäuses der Sprachsteuerungsmodul zu erkennen ist,
- Fig. 3: eine isometrische Darstellung eines Teils des Sprachsteuerungsmoduls nach Fig. 2, wobei das Innere des Gehäuses, in dem die den Schallsensor aufnehmende Platine angebracht ist, ersichtlich ist,
- Fig. 4: eine schematische Seitenansicht der Handgriffvorrichtung nach Fig. 1, wobei die Anordnung zwischen dem Sprachsteuerungsmodul und dem Griffelement zu erkennen ist,
- Fig. 5: eine Seitenansicht einer erfindungsgemäßen Operationsleuchte nach einem ersten vorteilhaften Ausführungsbeispiel, wobei die Handgriffvorrichtung wie schon in Fig. 4 schematisch dargestellt ist und an einem Leuch-tenaufnahmekörper der Operationsleuchte befestigt ist,
- Fig. 6: eine Unteransicht der in Fig. 5 dargestellten Operationsleuchte, wobei besonders gut die verschiedenen, jeweils eine Mehrzahl an Einzelleuchten aufweisenden Leuchtenfelder der Operationsleuchte, die im Betrieb das Wundfeld beleuchten, zu erkennen sind,
- Fig. 7: eine schematische Seitendarstellung einer Operationsleuchte mit zwei unterschiedlichen, in einem demontierten Zustand befindlichen Handgriffvor-richtungen, wobei die rechte der beiden dargestellten Handgriffvorrichtungen jene Handgriffvorrichtung der erfindungsgemäßen Art nach Fig. 1 bzw. 4 ist, und die linke der beiden Handgriffvorrichtungen eine aus dem Stand der Technik bekannte Handgriffvorrichtung ist, welche Handgriffvorrichtungen beide an den dargestellten Leuchtenaufnahmekörpern der Operationsleuchte befestigbar sind,
- Fig. 8: eine Operationsleuchte gemäß des Standes der Technik, d.h. mit einer Handgriffvorrichtung ohne dem erfindungsgemäßen Sprachsteuerungsmodul,
- Fig. 9: eine schematische Seitenansicht einer erfindungsgemäßen Operationsleuchte nach den Fign. 5 und 6, wobei besonders gut ein im Betrieb erzeugter Wirkbereich eines Abstandssensors des Sprachsteuerungsmoduls zu erkennen ist, und wobei sich im Wirkbereich des Abstandssensors ein Objekt, nämlich ein Kopf einer Person befindet,
- Fig. 10: eine schematische Seitenansicht eines Sprachsteuerungsmoduls für eine Handgriffvorrichtung nach einem vorteilhaften weiteren Ausführungsbeispiel, wobei das Sprachsteuerungsmodul nun ringförmig ausgebildet ist und keinen Aufnahmezapfen aufweist,
- Fig. 11: eine schematische Unteransicht der erfindungsgemäßen Operationsleuchte, wobei wie in Fig. 9 innerhalb der Reichweite des Sprachsteuerungsmoduls bzw. deren Abstandssensoren ein rundes Objekt, wie mit dem Pfeil gekennzeichnet, in die Lichtstrahlbündel einzelner Leuchtenfelder hinein bewegt wird, woraufhin die dunkel dargestellten Einzelleuchten der Leuchtenfelder aufgrund der detektierten Position mittels zumindest eines Abstandssensors selbsttätig mithilfe einer Rechnereinheit abgedimmt werden,
- Fig. 12: eine Unteransicht der Operationsleuchte gemäß Fig. 11, wobei das Objekt nun weiter in Richtung des zentralen Handgriffs / der zentralen Handgriffvorrichtung bewegt ist und gegenüber Fig. 11 weitere Einzelleuchten der Leuchtenfelder abgeschaltet sind,
- Fig. 13: wiederum eine Unteransicht der Operationsleuchte, gemäß Fign. 11 u. 12, wobei das Objekt gegenüber Fig. 12 noch weiter in Richtung des Zentrums, d.h. in Richtung der Handgriffvorrichtung bewegt ist und daraufhin noch weitere Einzelleuchten der Operationsleuchte abgedimmt / ausgeschaltet sind,
- Fig. 14: eine schematische Seitenansicht einer erfindungsgemäßen Operationsleuchte mit einer erfindungsgemäßen Handgriffvorrichtung nach einem weiteren vorteilhaften Ausführungsbeispiel, wobei nun innerhalb der Handgriffvorrichtung eine Bildaufzeichnungseinrichtung integriert ist,
- Fig. 15: eine Innenseite, d.h. eine dem Innenraum des Hohlkörperabschnittes zugewandte Seite, der in Fig. 3 bereits dargestellten Platine des Sprachsteue-rungsmoduls,
- Fig. 16: eine isometrische Detailansicht der Platine aus Fig. 15 im Bereich zweier benachbarter Abstandssensoren, wobei deren Anzeigeleuchten gut zu er-kennen sind,
- Fig. 17: eine isometrische Detailansicht einer Platine einer Handgriffvorrichtung gemäß einem weiteren vorteilhaften Ausführungsbeispiel, wobei deren Spracherkennungseinheit gut zu erkennen ist,
- Fig. 18: eine isometrische Darstellung einer Handgriffvorrichtung gemäß einem wei-teren vorteilhaften Ausführungsbeispiel, in die die Platine nach Fig. 17 ein-gesetzt ist,
- Fig. 19: eine isometrische Darstellung einer Handgriffvorrichtung gemäß einem weiteren vorteilhaften Ausführungsbeispiel, in die eine Bildaufzeichnungseinrichtung integriert ist, und
- Fig. 20: eine isometrische Darstellung der Handgriffvorrichtung nach Fig. 19, wobei das Gehäuse des Sprachsteuerungsmoduls nun nicht mehr transparent dargestellt ist.

Die Figuren sind lediglich schematischer Natur und dienen ausschließlich dem Verständnis der Erfindung. Die gleichen Elemente sind mit denselben Bezugszeichen versehen. Die Merkmale der unterschiedlichen Ausführungsbeispiele können auch frei miteinander kombiniert werden.

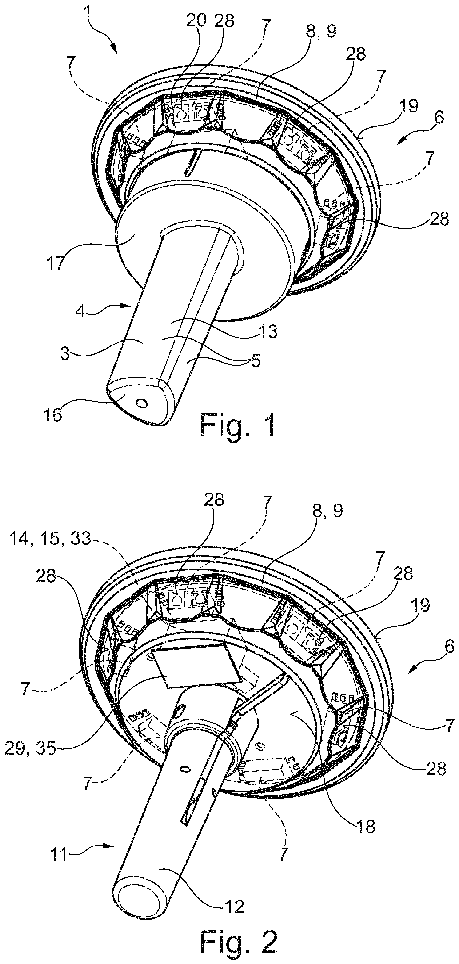

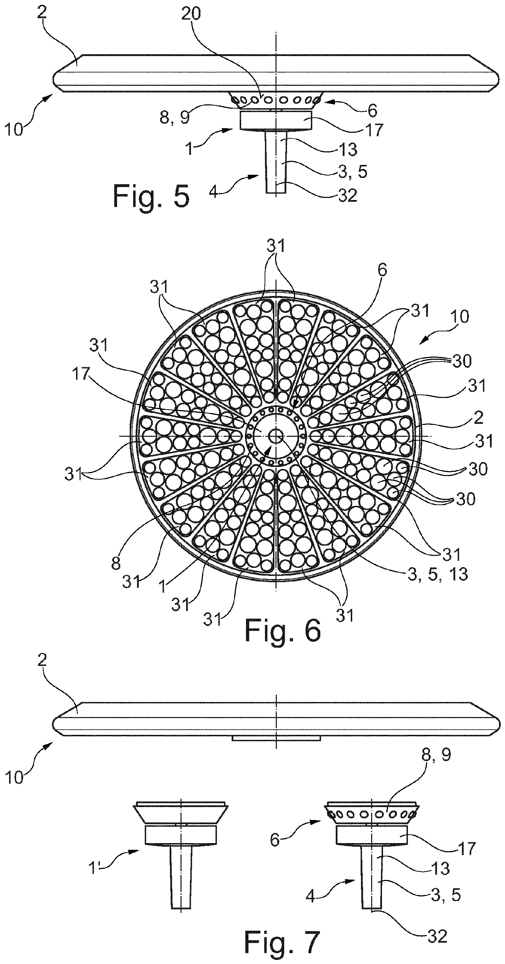

In Fig. 1 ist zunächst eine erfindungsgemäße Handgriffvorrichtung 1 nach einem vorteilhaften ersten Ausführungsbeispiel gut zu erkennen. Die Handgriffvorrichtung 1 ist für die Montage / für das vorübergehende Anbringen an einer nachfolgend in den Fign. 5 bis 9 sowie 11 bis 13 näher beschriebenen Operationsleuchte 10 vorbereitet. Die Erfindung betrifft somit nicht nur die Handgriffvorrichtung 1 selbst, sondern auch eine Operationsleuchte 10 mit einer solchen Handgriffvorrichtung 1.

Die Handgriffvorrichtung 1 weist ein, zum Anbringen an einem Leuchtenaufnahmekörper 2 der Operationsleuchte 10 vorbereitetes Griffelement 3 auf, welches Griffelement 3 auch schlicht als Handgriff bezeichnet ist. Das Griffelement 3 ist folglich derart dimensioniert, dass es durch eine Hand einer Person, wie einem Operateur, gegriffen werden kann, um die in einem Betriebszustand der Handgriffvorrichtung 1, in dem sie mit dem Leuchtenaufnahmekörper 2 der Operationsleuchte 10 fest verbunden ist, in die gewünschte Position zu bringen. Die nachfolgend (z.B. in Verbindung mit Fign. 5 und 6) noch näher beschriebene Operationsleuchte 10 weist weiterhin eine hier der Übersichtlichkeit halber nicht dargestellte Tragstruktur auf, die mit dem Leuchtenaufnahmekörper 2 verbunden ist und wodurch der Leuchtenaufnahmekörper 2 beliebig positionierbar ist. Auch weist die Tragstruktur einen elektrisch betätigbaren Antrieb auf, der hier der Übersichtlichkeit halber nicht weiter dargestellt ist und mit einer zentralen Steuereinheit der Operationsleuchte elektrisch verbunden ist.

An einem Außenbereich 4 des Griffelementes 3 ist eine Grifffläche 5 ausgebildet, die der Operateur im Betrieb zum Positionsändern der Operationsleuchte 10 berührt. Das Griffelement 3 an sich weist einen stabähnlichen, hohl ausgebildeten Hülsenabschnitt 13 auf, der sich länglich erstreckt. Unmittelbar an dessen Außenumfangseite / Au-ßenmantelseite ist jene Grifffläche 5 ausgebildet. Das Griffelement 3 ist an seinem Außenbereich 4 derart ausgebildet, dass es eine möglichst glatte Oberfläche / Grifffläche 5 aufweist, die leicht sterilisierbar ist. Das heißt, die Rauhigkeit der Grifffläche 5 ist derart gewählt, dass eine Reinigung mit anschließendem Sterilisieren einfach durchführbar ist und ein Fangen von Schmutzpartikeln vermieden ist.

Der Hülsenabschnitt 13 weist an einer Stirnseite, die im Betrieb dem Leuchtenaufnahmekörper 2 abgewandt ist, einen Deckel 16 auf, der das Innere des Hülsenabschnittes 13 zur Umgebung hin schützt / abschließt. Der Deckel 16 bildet daher einen ersten axialen Endbereich des Hülsenabschnittes 13 des Griffelementes 3 aus. Mit einem zweiten, dem ersten Endbereich gegenüberliegenden Endbereich geht der Hülsenabschnitt 13 in einen scheibenförmigen Verbreiterungsabschnitt 17 über. Verbreiterungsabschnitt 17 sowie Hülsenabschnitt 13 sind stoffeinteilig miteinander ausgeführt, d.h. einstückig miteinander verbunden.

Wiederum mit dem Griffelement 3 ist ein erfindungsgemäßes Sprachsteuerungsmodul 6 verbunden. Das Sprachsteuerungsmodul 6 ist wiederabnehmbar mit dem Griffelement 3 verbunden. Dabei ist das Sprachsteuerungsmodul 6 form- und kraftschlüssig mit dem Griffelement 3 verbunden. Das Sprachsteuerungsmodul 6, das gesamtheitlich in Fig. 2 in einem vom Griffelement 3 demontierten Zustand zu erkennen ist, weist wiederum ein Gehäuse 8 auf. Das Gehäuse 8 weist wiederum einen scheibenförmigen Hohlkörperabschnitt 9 auf bzw. ist durch diesen scheibenförmigen Hohlkörperabschnitt 9 ausgebildet. Der scheibenförmige Hohlkörperabschnitt 9 ist zur Umgebung hin abgedichtet. Der Hohlkörperabschnitt 9 ist zum einen durch zwei voneinander beabstandete Wände - einer Vorderwand 18 und einer Rückwand 19 - ausgebildet. Zum anderen weist der Hohlkörperabschnitt 9 eine ringförmig verlaufende Seitenwand 20 auf, die die Vorderwand 18 mit der Rückwand 19 verbindet. Die Vorderwand 18 ist dabei jene flach verlaufende Wand des Hohlkörperabschnittes 9, die im Betrieb dem Griffelement 3 zugewandt ist, wohingegen die Rückwand 19 Gehäuses jene flach verlaufende Wand des Hohlkörperabschnittes 9 ist, die im Betrieb dem Griffelement 3 abgewandt ist.

Weiterhin, weist das Sprachsteuerungsmodul 6, neben dem Gehäuse 8, einen Handgriffaufnahmeabschnitt 11 auf, der mit dem Gehäuse 8 / dem Hohlkörperabschnitt 9 fest verbunden ist. Dieser Handgriffaufnahmeabschnitt 11 ist dabei mit der Vorderwand 18 des Hohlkörperabschnittes 9 fest verbunden. Der Handgriffaufnahmeabschnitt 11 bildet einen Aufnahmezapfen 12 aus, der sich senkrecht von der, sich in einer gedachten Erstreckungsebene erstreckenden Vorderwand 18 des Hohlkörperabschnittes 9 weg erstreckt. Eine Längsachse 32 des Aufnahmezapfens 12 verläuft folglich senkrecht zu der Vorderwand 18 bzw. zu der Erstreckungsebene.

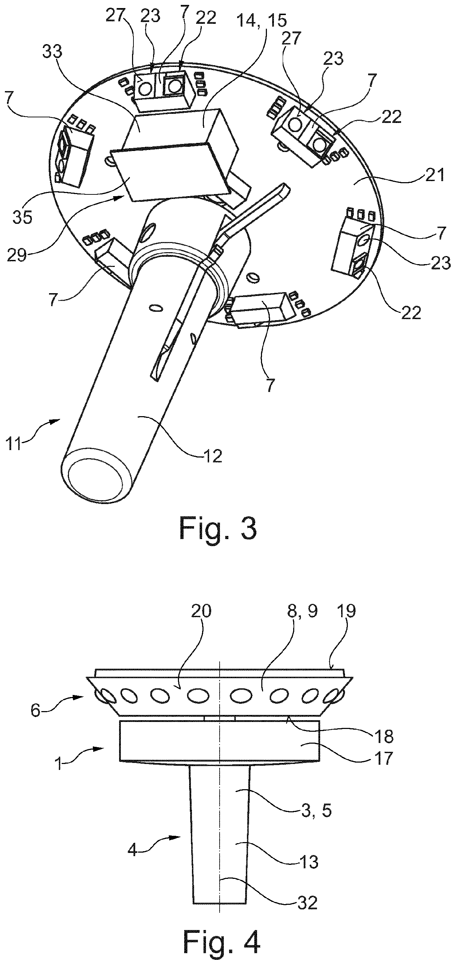

Der Aufnahmezapfen 12 ist derart dimensioniert und auf den Hülsenabschnitt 13 des Griffelementes 3 abgestimmt, dass er in den Hülsenabschnitt 13 einschiebbar ist. In dem in Fig. 1 dargestellten zusammengebauten Zustand der Handgriffvorrichtung 1 ist dieser Aufnahmezapfen 12 form- und kraftschlüssig in den Hülsenabschnitt 13 eingeschoben. Das Griffelement 3 ist daher form- und kraftschlüssig mit dem Sprachsteuerungsmodul 6 verbunden. Wie auch in der schematischen Darstellung nach Fig. 4 zu erkennen ist, ist der Verbreiterungsabschnitt 17 in diesem zusammengebauten Zustand in axialer Richtung des Aufnahmezapfens 12 (entspricht axialer Richtung der Längsachse 32 bzw. des Hülsenabschnittes 13) von der Vorderwand 18 distanziert gehalten.

In Fig. 3 ist schließlich der innere Aufbau des Sprachsteuerungsmoduls 6 veranschaulicht. In Fig. 3 ist der Übersichtlichkeit halber gegenüber der Fig. 2 das Gehäuse 8 / der Hohlkörperabschnitt 9 weggelassen, wodurch besonders gut die elektronische Einheit / der elektronisch Aufbau des Sprachsteuerungsmoduls 6 zu erkennen ist. Auf einer scheibenförmigen Platine 21 des Sprachsteuerungsmoduls 6 sind neben einem, einen Schallsensor 29 aufweisenden / umschließenden / aufnehmenden Elektronikgehäuse 33, mehrere Abstandssensoren 7 positioniert. Diese Abstandssensoren 7 sind alle gleich ausgebildet und funktionierend und weiter unten stehend näher erläutert.

Das Elektronikgehäuse 33 ist weiterhin mit einer auf der Platine 21 angeordneten Rechnereinheit 14 elektronisch verbunden. Das Elektronikgehäuse 33 nimmt die Rechnereinheit 14 sogar derart auf, dass diese von dem Elektronikgehäuse 33 umgeben ist. In dieser Ausführung ist die Rechnereinheit 14 folglich in dem Elektronikgehäuse 33 integriert. Neben der Rechnereinheit 14 ist in dem Elektronikgehäuse 33 auch der Schallsensor 29 aufgenommen, der auch als Mikrofon bezeichnet ist. Der Schallsensor 29 ist daher in Form einer Spracherkennungseinheit 15 (auch als Spracherkennungsmodul oder Sprachsteuerungseinheit bezeichnet) in dem Elektronikgehäuse 33 integriert. Der Schallsensor 29 ist elektrisch mit der Rechnereinheit 14 verbunden. Somit werden in einem im Betrieb mit der Operationsleuchte 10 verbundenen Zustand der Handgriffvorrichtung 1 Steuersignale in Form von Schallsignalen durch den Schallsensor 29 detektiert / aufgenommen und in elektrischer Form an die Rechnereinheit 14 weitergeleitet. Durch die Rechnereinheit 14, die wiederum in diesem Betriebszustand mit einer zentralen Steuereinheit der Operationsleuchte 10, die hier der Übersichtlichkeit halber nicht weiter dargestellt ist, elektrisch verbunden ist, können somit Steuerbefehle an die zentrale Steuereinheit übertragen werden. Dadurch ist die Operationsleuchte 10 individuell mit einzelnen durch den Schallsensor 29 aufgenommenen Sprachbefehlen bedienbar.

Neben der Bedienung der Helligkeit / Beleuchtungsstärke der jeweiligen Einzelleuchten 30 der Operationsleuchte 10, ist es auch möglich bspw. eine Stellung der Operationsleuchte 10 durch Betätigen eines auf die Tragstruktur der Operationsleuchte 10 einwirkenden elektrischen Antriebes mittels dieser Sprachbefehle zu verändern. Die Spracherkennungseinheit 15 weist weiterhin auch einen Lautsprecher auf, der ebenfalls in dem Elektronikgehäuse 33 integriert ist.

Wie in Fig. 2 zu erkennen ist, weist die Vorderwand 18 des Hohlkörperabschnittes 9 eine auf das Elektronikgehäuse 33 bzw. auf den Schallsensor 29 abgestimmte Aussparung auf, in die eine Außenabdeckung 35 / Membran, die schallübertragend ausgebildet ist, eingesetzt ist. In den Fign. 2 und 3 überragt eine schalldurchlässige Außenabdeckung 35 des Mikrofons 29 zu allen Seiten hin das Elektronikgehäuse 33. Folglich ist eine Schallübertragung durch die Vorderwand 18 hindurch, in das Innere des Gehäuses 8 und des Elektronikgehäuses 33, zum Schallsensor 29 hin, ermöglicht.

In einer weiteren Ausführungsform ist die Recheneinheit 14 nicht wie hier kabelgebunden mit der zentralen Steuereinheit der Operationsleuchte 10 verbunden, sondern drahtlos, über eine Bluetooth-Datenverbindung.

Dadurch sind entsprechend der ermittelten Position des Objektes 25 die einzelnen Leuchten / Einzelleuchten 30 der Leuchtenfelder 31 der Operationsleuchte 10, die das Objekt 25 bescheinen, abgedimmt oder entsprechend abgeschaltet werden können.

Im Weiteren ist gemäß dem ersten Ausführungsbeispiel jeder der Abstandssensoren 7 mit der auf der Platine 21 angeordneten Rechnereinheit 14 elektronisch verbunden. Die Abstandssensoren 7 sind als Infrarotsensoren (auch als Infrarotdistanzsensoren bezeichnet) ausgebildet. Jeder Abstandssensor 7 weist dabei einen im Wesentlichen tropfenförmigen / ballonförmigen Wirkbereich 24 auf, innerhalb dessen ein Objekt 25, wie ein Kopf des Operateurs detektierbar ist. Mit einem Infrarotsender 22 des Abstandssensors 7 wird hierfür Infrarotlicht ausgesandt, welches Infrarotlicht sich im Wesentlichen trichter-/kegelförmig entlang einer gedachten Richtachse 26 räumlich ausbreitet. Neben dem Infrarotsender 22 weist der Abstandssensor 7 auch einen Infrarotnehmer 23 auf. Der Infrarotnehmer 23 ist so ausgelegt und ausgebildet, dass er, wenn sich ein Objekt 25 innerhalb des Wirkbereiches 24 befindet, einen durch das Objekt 25 reflektierten Anteil des zuvor durch den Infrarotsender 22 ausgesandten Infrarotlicht messtechnisch erfasst / detektiert.

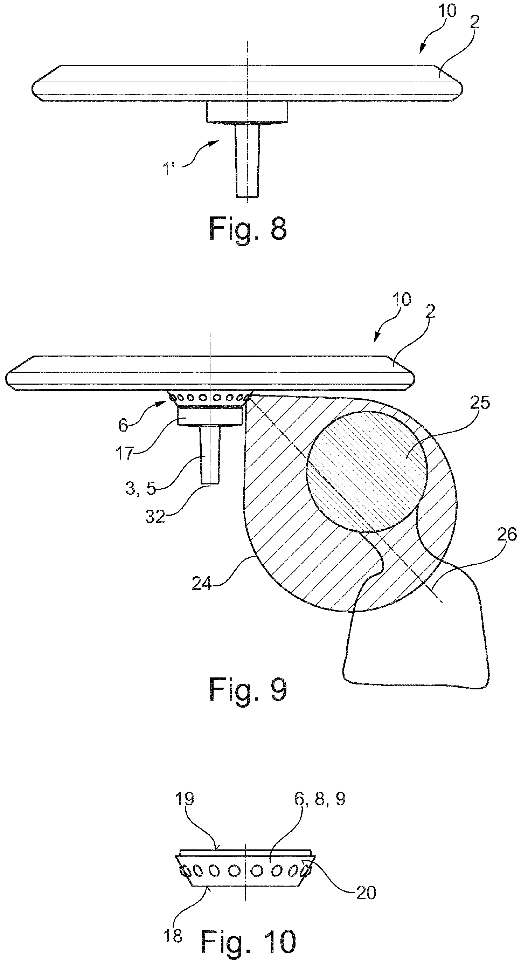

Der Wirkbereich 24 ist besonders gut in Fig. 9 zu erkennen, wobei in dieser Abbildung die Handgriffvorrichtung 1 bereits an der Operationsleuchte 10 montiert ist. Der an dem Objekt 25 reflektierte Anteil an Infrarotlicht wird daher dem Infrarotnehmer 23 zugeführt, der das reflektierte Infrarotlicht in ein Steuersignal umsetzt. In Abhängigkeit der Entfernung des Objektes 25 vom Abstandssensor 7 / vom Infrarotnehmer 23, detektiert der Infrarotnehmer 23 das Signal zu einem früheren oder einem späteren Zeitpunkt relativ zu dem Absendezeitpunkt des Infrarotlichtes durch den Infrarotsender 22. Dadurch ist der Abstand zwischen dem Objekt 25 und der Handgriffvorrichtung 1 einfach detektierbar. Der Wirkbereich 24 ist folglich durch die Form des ausgesandten Infrarotlichtes sowie die Reichweite des Infrarotnehmers 23 beschränkt. Jeder Abstandssensor 7 ist daher ausgebildet, eine Position des Objektes 25 in einem Bereich zwischen dem Lampenaufnahmegehäuse 2 und einem Wundfeld über den Abstand des Objektes 25 relativ zu dem Abstandssensor 7, bzw. zu dem Sprachsteuerungsmodul 6, zu detektieren / zu ermitteln. Aufgrund der verschiedenen Sensoren - Schallsensor 29 sowie Abstandssensoren 7 - ist das Sprachsteuerungsmodul 6 auch als Sensormodul bezeichnet.

Die Abstandssensoren 7 sind auf der Platine 21 derart angebracht, dass ihr Wirkbereich 24 mit seiner Richtachse 26 quer / schräg zu der Längsachse 32 des Aufnahmezapfens 12, insbesondere vorzugsweise um etwa 45° versetzt zu dieser Längsachse 32 ausgerichtet ist. Der Infrarotsender 22 und der Infrarotnehmer 23 sind in einer Aufnahmeebene 27 angeordnet / ausgerichtet, die senkrecht zu der Richtachse 26 ausgerichtet ist.

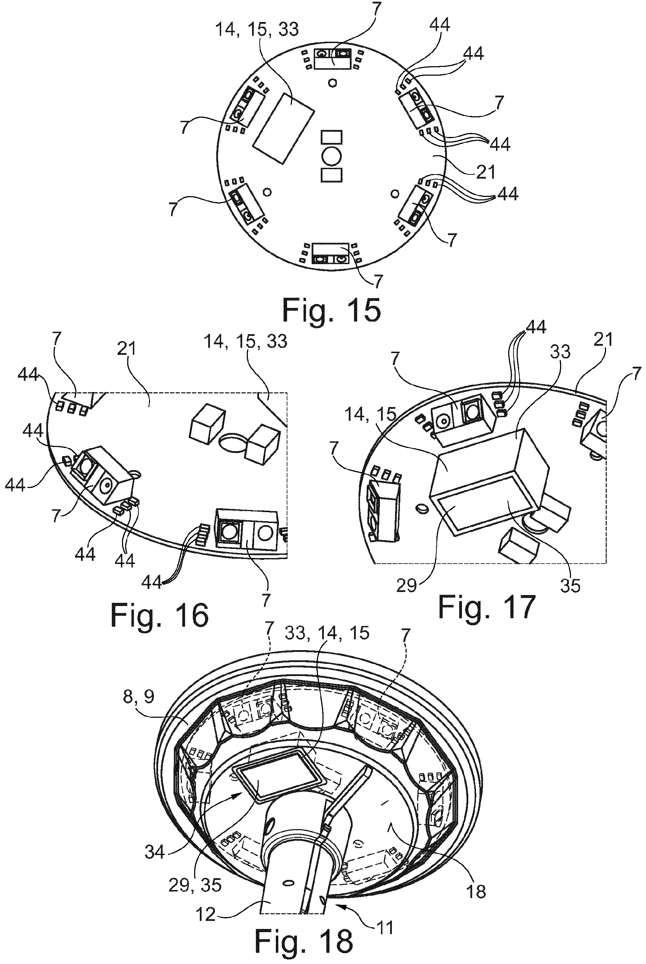

In dieser Ausführungsform sind sechs Abstandssensoren 7 im Wesentlichen entlang einer kreisförmigen, gedachten Umfangslinie um die Längsachse des Aufnahmezapfens 12 herum verteilt angeordnet, wobei jedoch auch in weiteren Ausführungsformen andere Stückzahlen von Abstandssensoren 7 gewählt sind, bspw. weniger oder mehr als sechs, vorzugsweise sieben, acht, neun oder mindestens zehn Abstandssensoren 7. Die Abstandssensoren 7 sind jeweils in gleichen Abständen entlang der gedachten Umfangslinie angeordnet und haben einen im Wesentlichen gleichen Abstand zu der Längsachse 32 des Aufnahmezapfens 12. Die Abstandssensoren 7 sind dabei derart angeordnet und ausgerichtet, dass sie mit ihren Wirkbereichen 24 die Position eines Objektes 25 innerhalb des gesamten Umfangs, d.h. in einem Winkelbereich von 360° um die Längsachse 32 herum, erfassen können. In der Fig. 15 ist auch nochmals die Verteilung der Abstandssensoren 7 schematisch dargestellt, wobei die Rechnereinheit 14 in dieser Ansicht der Übersichtlichkeit halber kein Mikrofon 29 aufweist.

Zur Anzeige ob ein Objekt 25 in dem Wirkbereich 24 des jeweiligen Abstandssensors 7 befindlich ist, sind weiterhin auf der Platine 21 mehrere Anzeigeleuchten 44 je Abstandssensor 7 angebracht, die besonders gut in Fig. 16 zu erkennen sind. Wenn ein Objekt 25 in dem Wirkbereich 24 des jeweiligen Abstandssensors 7 befindlich ist, erhöht sich die Anzahl an leuchtenden / eingeschalteten Anzeigeleuchten 44 mit einem sich verringernden Abstand zwischen Abstandssensor 7 und Objekt 25. In dieser Ausführung sind je Abstandssensors 7 sechs Anzeigeleuchten 44 vorhanden. Jede Anzeigeleuchte 44 ist als eine LED ausgebildet. Auch ist es möglich in Abhängigkeit des Abstandes zwischen Abstandssensor 7 und Objekt 25 zwischen verschiedenen Anzeigeleuchte 44, die sich in ihrer Leuchtfarbe unterscheiden, hin- und herzuschalten.

Wie besonders gut auch in den Fign. 1 und 2 zu erkennen ist, ist die Seitenwand 20 des Gehäuses 8 mit mehreren Abdeckungsbereichen 28 ausgebildet. Jeder Abdeckungsbereich 28 erstreckt sich parallel zu der Aufnahmeebene 27 und daher konisch zwischen der Vorderwand 18 und der Rückwand 19. Die kreisrunde Vorderwand 18 weißt einen geringeren Durchmesser auf als die ebenfalls kreisrund ausgebildete Rückwand 19. Die Seitenwand 20 ist in dieser Ausführung aus einem Material hergestellt, das durchlässig für die Messsignale der Abstandssensoren 7 ist. Folglich ist diese Seitenwand 20 durchlässig für Infrarotlicht. Da das konisch ausgeformte Gehäuse 8 / der konisch ausgeformte Hohlkörperabschnitt 9 gesamtheitlich aus dem gleichen Werkstoff besteht, ist dieser gesamtheitlich aus einem Infrarotlichtdurchlässigen Material / Werkstoff ausgebildet. Neben der infrarotdurchlässigen Seitenwand 20, ist somit auch die Vorderwand 18 infrarotlichtdurchlässig. Jedem Abstandssensor 7 ist folglich ein parallel zu dessen Aufnahmeebene 27 verlaufender Abdeckungsbereich 28 zugeordnet, der sich im Wesentlichen um die gleiche Breite erstreckt, wie der jeweilige Abstandssensor 7.

In den Fign. 5 und 6 ist dann besonders gut auch eine erfindungsgemäße Operationsleuchte 10 mit einer montierten Handgriffvorrichtung 1 gemäß der Fign. 1 bis 4 zu erkennen. Die Operationsleuchte 10 weist dafür den Leuchtenaufnahmekörper 2, der auch als Grundkörper bezeichnet ist, auf, in dem mehrere Einzelleuchten 30 eingesetzt sind. Die einzelnen Einzelleuchten 30 sind in dieser Ausführungsform mit weiteren Einzelleuchten 30 zu unterschiedlichen Leuchtenfeldern 31 kombiniert / gruppiert. Die Operationsleuchte 10 weist in dieser Ausführung einen im Wesentlichen scheibenförmigen und gehäuseartigen Leuchtenaufnahmekörper 2 auf, der in weiteren Ausführungen jedoch auch andere Ausgestaltungen aufweist und bspw. auch mehrteilig, d.h. aus mehreren Leuchtenaufnahmekörper-Segmenten, ausbildbar ist. Die Leuchtenfelder 31 (auch als Leuchtfelder bezeichnet) sind jeweils im Wesentlichen gleich funktionierend, angesteuert und aufgebaut.

Jedes Leuchtenfeld 31 weist die gleiche Anzahl an Einzelleuchten 30 auf. Die Einzelleuchten 30 eines Leuchtenfeldes 31 variieren dabei in ihrer Größe und/oder Helligkeit / Leuchtstärke / Beleuchtungsstärke. Auch die Leuchtfarbe der Einzelleuchten 30 untereinander variiert hierbei. Jedes Leuchtenfeld 31 ist dabei in Form eines tortenförmigen Stückes der sich scheibenförmig um die zentrale Handgriffvorrichtung 1 herum erstreckenden Gesamtzahl von Einzelleuchten 30 ausgebildet. Jede der Einzelleuchten 30 umfasst dabei ausschließlich eine LED, in weiteren Ausführungen jedoch auch mehrere LEDs. Jede Einzelleuchte 30 weißt eine der LED zugeordnete Linse / Lindenoptik auf. Jede der Einzelleuchten 30 ist dabei mit der zentralen Steuereinheit der Operationsleuchte 10 elektrisch verbunden und in Abhängigkeit der Steuersignale seitens der zentralen Steuereinheit unabhängig voneinander regelbar, insbesondere in ihrer Leuchtstärke / Leuchtfarbe regelbar.

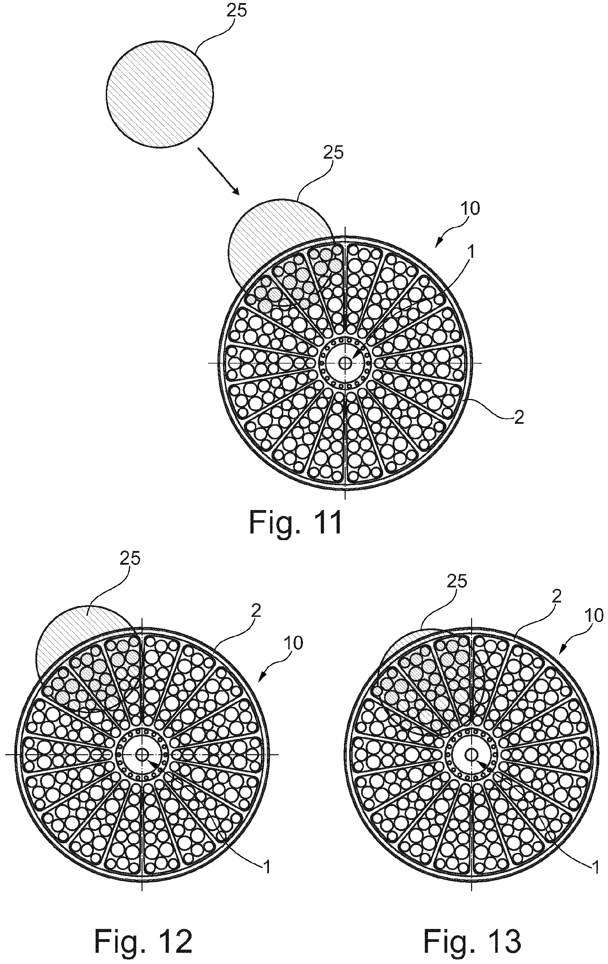

Wie im Zusammenhang mit den Fign. 9 und 11 bis 13 zu erkennen ist, ist im Betrieb der Operationsleuchte 10 ein Überwachen eines Bereiches, der durch die Einzelleuchten 30 ausgeleuchtet wird, mittels der Abstandssensoren 7 umgesetzt. Die einzelnen Abstandssensoren 7 wirken dabei vorzugsweise in einer Reichweite von mehr als einem, vorzugsweise von mehr als zwei Metern in Richtung der Richtachse 26. Dadurch wird aufgrund eines in den Lichtstrahl / in das Lichtstrahlbündel einer Einzelleuchte 30 tretenden Objektes 25 gemäß der Fign. 11 bis 13 durch die Abstandssensoren 7 der Abstand zwischen dem Objekt 25 und dem Sprachsteuerungsmodul 6 erfasst / detektiert und daraufhin ein Steuersignal durch die Rechnereinheit 14 erzeugt, wodurch mittels der zentralen Steuereinheit die Einzelleuchten 30 der betroffenen Leuchtenfelder 31 (d.h. die Einzelleuchten 30, die mit ihren Lichtstrahlbündel das Objekt direkt beleuchten) herunter gedimmt bzw. abgeschaltet werden. Ein Abdimmen erfolgt hierbei je nach Höhenabstand zwischen der Operationsleuchte 10 und dem Objekt 25. Wie in den Fign. 11 bis 13 gut zu erkennen ist, können dabei die Leuchtenfelder 31 gemäß Fig. 13 gesamtheitlich oder die Einzelleuchten 30 der Leuchtenfelder 31 einzeln, je nach Position des Objektes 25 relativ zu der Handgriffvorrichtung 1 / zum Abstandssensor 7, abgeschaltet / gedimmt werden.

Die Handgriffvorrichtung 1 ist in ihrem montierten Zustand zentral an dem Leuchtenaufnahmekörper 2 befestigt und somit mit der Längsachse 32 koaxial zu einer gedachten Mittelachse des Leuchtenaufnahmekörpers 2 angeordnet.

Im Weiteren hat auch der Schallsensor 29 eine Reichweite von mindestens einem, besonders bevorzugt von mindestens zwei Metern, wodurch Schallquellen, die ein entsprechendes Schallsteuersignal erzeugen, etwa das Objekt 25 selbst, detektiert werden können, um die Operationsleuchte 10 entsprechend anzusteuern.

In den Fign. 7 und 8 wird dann nochmals besonders gut die vorteilhafte Ausbildung der Handgriffvorrichtung 1 sowie die Zusammenwirkung mit der Operationsleuchte 10 erkennbar. So ist es ein Einfaches, eine herkömmliche Handgriffvorrichtung 1' nach dem Stand der Technik, wie sie in Fig. 8 bzw. auf der linken Seite in Fig. 7 dargestellt ist, durch eine erfindungsgemäße Handgriffvorrichtung 1, wie sie auf der rechten Seite in Fig. 7 dargestellt ist, auszutauschen, indem der wieder abnehmbare Teil der alten Handgriffvorrichtung 1' von der Operationsleuchte 10 entfernt wird und die erfindungsgemäße Handgriffvorrichtung 1 an dem Leuchtenaufnahmekörper 2 befestigt wird. Zu diesem Zwecke bildet das Sprachsteuerungsmodul 6 an der Rückwand 19 des Hohlkörperabschnittes 9 einen wiederabnehmbaren Abschnitt aus, der wiederabnehmbar mit dem Leuchtenaufnahmekörper 2 verbunden wird. Zu diesem Zwecke ist der Hohlkörperabschnitt 9 form- und kraftschlüssig, vorzugsweise mittels einer Art Bajonettverschluss wiederabnehmbar / lösbar mit dem Leuchtenaufnahmekörper 2 verbunden. Dadurch ist dann die fertig montierte Stellung der Operationsleuchte 10 bzw. der Handgriffvorrichtung 1 umgesetzt, wie es etwa in Fig. 9 dargestellt ist.

In Verbindung der Fign. 17 und 18 ist ein weiteres Ausführungsbeispiel der erfindungsgemäßen Handgriffvorrichtung 1 schematisch dargestellt, wobei diese Handgriffvorrichtung 1 gemäß dem ersten Ausführungsbeispiel funktioniert und ausgebildet ist. Als Unterschied ist hier die Außenabdeckung 35 des Schallsensors 29 in einen durch das Elektronikgehäuse 33 gebildeten Rahmen eingesetzt und dadurch besonders geschickt formschlüssig in dem Elektronikgehäuse 33 gehalten. In Fig. 18 ist dabei zu erkennen, dass in dem Gehäuse 8 des Sensormoduls 6, nämlich in dessen Vorderwand 18, eine mit dem Elektronikgehäuse 33 korrespondierende Aussparung 34 ausgebildet ist, in die das Elektronikgehäuse 33 seitens der Außenabdeckung 35 hineinragt.

In Verbindung mit Fig. 10 ist dann noch ein weiteres Ausführungsbeispiel der erfindungsgemäßen Handgriffvorrichtung 1 offenbart, wobei in erster Linie ein weiteres Sprachsteuerungsmodul 6 in einer alternativen Ausführung zu erkennen ist. Das Sprachsteuerungsmodul 6 besteht hierbei nur aus dem zuvor beschriebenen, scheibenförmigen Hohlkörperabschnitt 9 in Form eines Zwischenrings, der das Gehäuse 8 ausbildet. Ein als Aufnahmezapfen 12 ausgebildeter Handgriffaufnahmeabschnitt 11 ist hierbei weggelassen. In dieser Ausführungsform ist es umgesetzt, dass eine bestehende Handgriffvorrichtung 1 direkt mit dem Gehäuse 8 wieder abnehmbar verbunden ist. Auch dadurch ist es daher dann möglich, ein wieder abnehmbares Sprachsteuerungsmodul 6 zur Verfügung zu stellen. Dessen Aufbau sowie Funktionsweise entspricht hierbei dem Sprachsteuerungsmodul 6 des ersten Ausführungsbeispieles.

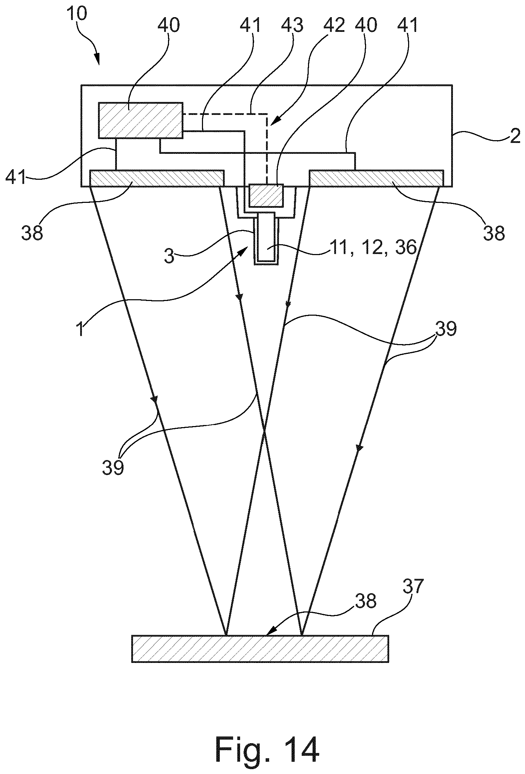

Zudem ist in Fig. 14 noch ein weiteres Ausführungsbeispiel der erfindungsgemäßen Handgriffvorrichtung 1 schematisch dargestellt, wobei die Handgriffvorrichtung 1 wiederum bereits an dem Leuchtenaufnahmekörper 2 befestigt ist. Die Handgriffvorrichtung 1 entspricht hierbei im Wesentlichen der Handgriffvorrichtung 1 des ersten Ausführungsbeispiels. Auch die Operationsleuchte 10 entspricht hierbei im Wesentlichen der Operationsleuchte 10, wie sie in Zusammenhang mit den Fig. 5, 6, 9 und 11 bis 13 beschrieben ist. Die die Leuchtenfelder 31 bildenden Einzelleuchten 30 der Operationsleuchte 10 sind hierbei schematisch als Rechtecke dargestellt, die jeweils mehrere Lichtstrahlen / Lichtstrahlbündel erzeugen, um somit etwa auf einer im Wundfeld positionierten Ausleuchtungsebene 37 ein gemeinsames Lichtfeld 38 / Ausleuchtfeld auszubilden.

Schematisch ist hierbei auch besonders gut die zuvor allgemein beschriebene zentrale Steuereinheit der Operationsleuchte 10 zu erkennen, welche Steuereinheit nun mit dem Bezugszeichen 40 gekennzeichnet ist. Die Steuereinheit 40 ist in dem Leuchtenaufnahmekörper 2 angeordnet. Die Steuereinheit 40 ist mit jedem Leuchtenfeld 31 über elektrische Verbindungsleitungen 41 elektrisch verbunden. Innerhalb der Leuchtenfelder 31 spaltet sich die Verbindungsleitung 41 wiederum so auf, dass jede Einzelleuchte 30 mit einer Nebenleitung elektrisch mit der Verbindungsleitung 41 verbunden ist.

Auch ist das hier schematisch dargestellte Sprachsteuerungsmodul 4 als "Stand-Alone"-Modul ausgebildet und mit der Steuereinheit 40 mittels einer Datenverbindung 42 verbunden. Die Datenverbindung 42 erfolgt in dieser Ausführungsform durch eine, hier gestrichelt dargestellten Datenverbindungsleitung 43, d.h. elektrisch / kabelgebunden. In einer weiteren Ausführungsform ist diese Datenverbindung 42 auch als eine drahtlose Verbindung ausgebildet.

Im Unterschied zu dem ersten Ausführungsbeispiel, umfasst die Handgriffvorrichtung 1 jedoch weiterhin eine Bildaufzeichnungseinrichtung 34 umfassend eine Kamera, nämlich eine Videokamera zum Filmen des Wundfeldes / der Ausleuchtungsebene 37. Die Bildaufzeichnungseinrichtung 34 weist zudem Tonaufzeichnungsmittel zum Aufnehmen eines Tons sowie einen Lautsprecher auf, die hier der Übersichtlichkeit halber nicht dargestellt sind. Die Bildaufzeichnungseinrichtung 34 ist hierbei innerhalb, d.h. radial innerhalb des Griffelementes 3 angeordnet. Die Bildaufzeichnungseinrichtung 34 ist weiterhin in dem Handgriffaufnahmeabschnitt 11 / dem Aufnahmezapfen 12 integriert. Die Videokamera der Bildaufzeichnungseinrichtung 34 ist hierbei mit ihrer Linse zu einer dem Leuchtenaufnahmekörper 2 abgewandten Seite der Handgriffvorrichtung 1 ausgerichtet. Zu diesem Zwecke ist der Deckel 16 vorzugsweise weggelassen oder transparent für Licht, das durch die Videokamera wahrnehmbar ist, ausgebildet. Ebenfalls in der Bildaufzeichnungseinrichtung 34 in der Handgriffvorrichtung 1 ist eine Speichereinheit in Form einer Speicherkarte integriert, die mit der Videokamera verbunden ist und zum Speichern der durch die Videokamera aufgenommen Daten ausgebildet ist.

Die Bildaufzeichnungseinrichtung 34 ist wiederum mit der Steuereinheit 40 im Betriebszustand gemäß Fig. 14 mit einer weiteren Verbindungsleitung 41 elektrisch verbunden. Die Stromversorgung der Bildaufzeichnungseinrichtung 34, der Spracherkennungseinheit 15 und der Rechnereinheit 14 erfolgt im Betrieb mittels dieser Verbindungsleitung 41. Auch die Abstandssensoren 7 sind mittels dieser Verbindungsleitung 41 im Betrieb bestromt.

Auch ist die Bildaufzeichnungseinrichtung 34 mit dem Sprachsteuerungsmodul 4 elektrisch verbunden. Dadurch ist die Bildaufzeichnungseinrichtung 34 durch das Sprachsteuerungsmodul 4 direkt betätigbar, nämlich ein- bzw. ausschaltbar. Dadurch lässt sich einer Aufnahme der Videokamera ebenfalls mittels Spracheingabe / Sprachbefehl starten bzw. anhalten. Auch ist es hierdurch ermöglicht eine Sprachmemo-Aufnahme, mittels des Tonaufzeichnungsmittels direkt auf der Speicherkarte abzuspeichern. Die durch die Bildaufzeichnungseinrichtung 34 erfassten Daten werden dann im Betrieb mittels der Datenverbindung 42 an die Steuereinheit 40 übermittelt und von dieser weitergeleitet oder weiterverarbeitet.

Die Verbindungsleitungen 41 und die Datenverbindung 42 sind hierbei Bestandteil eines Kabel-Bus-Systems, nämlich eines CAN-Bus-Systems.

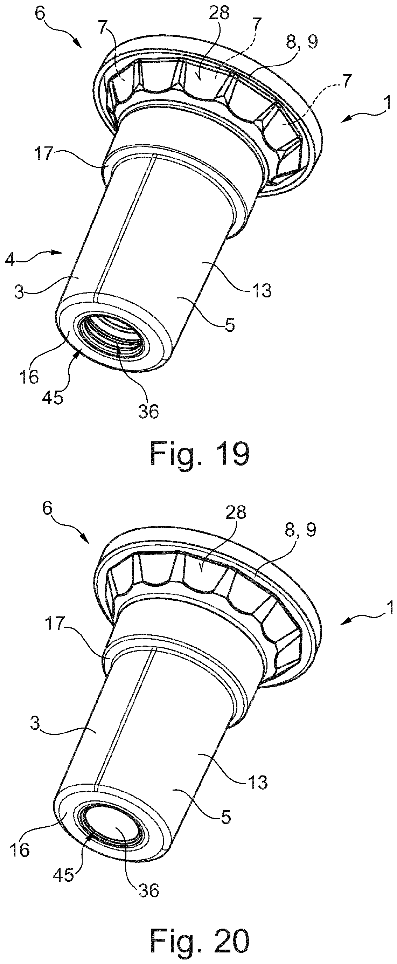

In den Fign. 18 und 19 ist noch ein weiteres Ausführungsbeispiel der erfindungsgemäßen Handgriffvorrichtung 1 dargestellt, wobei diese Handgriffvorrichtung 1 gemäß dem Ausführungsbeispiel nach Fig. 14 funktioniert und ausgebildet ist. Insbesondere ist hierbei jedoch das Griffelement 3 dicker ausgebildet. Dies liegt daran, dass in dem Griffelement 3 eine die Bildaufzeichnungseinrichtung 36 ausbildende Videokamera zur Aufnahme des Wundfeldes angeordnet ist. Von der Bildaufzeichnungseinrichtung 36 ist die äußerte Scheibe / das äußerste Glas deren Objektives zu erkennen, wobei deren Lichteintrittsachse konzentrisch zu einem Durchgangsloch 38 ausgebildet ist. Die Bildaufzeichnungseinrichtung 36 ist von der Grifffläche 5 umgeben, jedoch im Bereich des Deckels 16 mit dem Durchgangsloch 38 versehen, durch die die Bildaufzeichnungseinrichtung 36 das Äußere der Handgriffvorrichtung 1 erfasst.

### Bezugszeichen

- 1: Handgriffvorrichtung
- 1': Handgriffvorrichtung nach dem Stand der Technik
- 2: Leuchtenaufnahmekörper
- 3: Griffelement
- 4: Außenbereich
- 5: Grifffläche
- 6: Sprachsteuerungsmodul / Sensormodul
- 7: Abstandssensor
- 8: Gehäuse
- 9: Hohlkörperabschnitt
- 10: Operationsleuchte
- 11: Handgriffaufnahmeabschnitt
- 12: Aufnahmezapfen
- 13: Hülsenabschnitt
- 14: Recheneinheit
- 15: Spracherkennungseinheit
- 16: Deckel
- 17: Verbreiterungsabschnitt
- 18: Vorderwand
- 19: Rückwand
- 20: Seitenwand
- 21: Platine
- 22: Infrarotsender
- 23: Infrarotnehmer
- 24: Wirkbereich
- 25: Objekt
- 26: Richtachse
- 27: Aufnahmeebene
- 28: Abdeckungsbereich
- 29: Schallsensor / Mikrofon
- 30: Einzelleuchte
- 31: Leuchtenfeld
- 32: Längsachse
- 33: Elektronikgehäuse
- 35: Außenabdeckung
- 36: Bildaufzeichnungseinrichtung
- 37: Ausleuchtungsebene
- 38: Lichtfeld
- 39: Lichtstrahl
- 40: Steuereinheit
- 41: Verbindungsleitung
- 42: Datenverbindung
- 43: Datenverbindungsleitung

## Patentansprüche

1. Handgriffvorrichtung (1), die zum Anbringen an einem Leuchtenaufnahmekörper (2) einer Operationsleuchte (10) vorbereitet ist, mit einem Griffelement (3), das an einem Außenbereich (4) eine Grifffläche (5) ausbildet, wobei mit dem Griffelement (3) ein zumindest einen Schallsensor (29) aufweisendes Sprachsteuerungsmodul (6) wieder abnehmbar verbunden ist, wobei das Sprachsteuerungsmodul (6) weiterhin eine Rechnereinheit (14) aufweist, welche Rechnereinheit (14) elektrisch mit dem zumindest einen Schallsensor (29) verbunden ist, **dadurch gekennzeichnet, dass** die Rechnereinheit (14) zum Erzeugen von Steuersignalen zum Steuern der Operationsleuchte (10) in Abhängigkeit von den mittels dem zumindest einen Schallsensor (29) ermittelten Messdaten ausgebildet ist.

2. Handgriffvorrichtung (1) nach Anspruch 1, **dadurch gekennzeichnet, dass** das Sprachsteuerungsmodul (6) ein Gehäuse (8) aufweist, in dem der zumindest eine Schallsensor (29) aufgenommen ist.

3. Handgriffvorrichtung (1) nach Anspruch 2, **dadurch gekennzeichnet, dass** das Gehäuse (8) einen scheibenförmigen Hohlkörperabschnitt (9) aufweist, innerhalb dessen der zumindest eine Schallsensor (29) angeordnet ist.

4. Handgriffvorrichtung (1) nach Anspruch 2 oder 3, **dadurch gekennzeichnet, dass** das Gehäuse (8) im Bereich des zumindest einen Schallsensors (29) durchlässig für ein durch den zumindest einen Schallsensor (29) zu detektierendes Schallsignal ausgebildet ist.

5. Handgriffvorrichtung (1) nach einem der Ansprüche 1 bis 4, **dadurch gekennzeichnet, dass** das Sprachsteuerungsmodul (6) einen Handgriffaufnahmeabschnitt (11) aufweist, der wiederabnehmbar mit dem Griffelement (3) verbunden ist.

6. Handgriffvorrichtung (1) nach Anspruch 5, **dadurch gekennzeichnet, dass** der Handgriffaufnahmeabschnitt (11) einen Aufnahmezapfen (12) ausbildet, auf dem in zumindest einem Betriebszustand der Handgriffvorrichtung (1) ein, die Grifffläche (5) aufweisender Hülsenabschnitt (13) des Griffelementes (3) aufgesteckt ist.

7. Handgriffvorrichtung (1) nach einem der Ansprüche 1 bis 6, **dadurch gekennzeichnet, dass** das Griffelement (3) form- und/oder kraftschlüssig mit dem Sprachsteuerungsmodul (6) in zumindest einem Betriebszustand verbunden ist.

8. Handgriffvorrichtung (1) nach einem der Ansprüche 1 bis 7, **dadurch gekennzeichnet, dass** in dem Sprachsteuerungsmodul (6) zumindest ein Abstandssensor (7) enthalten ist, der zum Detektieren einer Position eines Objektes (25) ausgelegt ist.

9. Handgriffvorrichtung (1) nach Anspruch 8, **dadurch gekennzeichnet, dass** der zumindest eine Abstandssensor (7) elektronisch mit der Rechnereinheit (14) verbunden ist.

10. Handgriffvorrichtung (1) nach einem der Ansprüche 1 bis 9, **dadurch gekennzeichnet, dass** das Sprachsteuerungsmodul (6) mit einer Bildaufzeichnungseinrichtung verbunden ist.

11. Operationsleuchte (10) mit einer Handgriffvorrichtung (1) nach einem der Ansprüche 1 bis 10, wobei die Handgriffvorrichtung (1) zumindest teilweise wieder abnehmbar mit einem Leuchtenaufnahmekörper (2) der Operationsleuchte (10) verbunden ist.

12. Operationsleuchte (10) nach Anspruch 11, **dadurch gekennzeichnet, dass** das Sprachsteuerungsmodul (6) wieder abnehmbar an dem Leuchtenaufnahmekörper (2) angebracht ist.

13. Operationsleuchte (10) nach Anspruch 11 oder 12, **dadurch gekennzeichnet, dass** die Rechnereinheit (14) des Sprachsteuerungsmoduls (6) drahtgebunden mit einer zentralen Steuereinheit (40) der Operationsleuchte (10) verbunden ist.

14. Operationsleuchte (10) nach Anspruch 11 oder 12, **dadurch gekennzeichnet, dass** die Rechnereinheit (14) des Sprachsteuerungsmoduls (6) mittels einer drahtlosen Datenverbindung mit einer zentralen Steuereinheit (40) der Operationsleuchte (10) verbunden ist.

## Claims

1. A handle device (1) which is prepared for attachment to a receiving body (2) of a surgical light (10), which comprises a handle element (3) forming a handle surface (5) in an exterior region (4), wherein a voice control module (6) having at least one acoustic sensor (29) is detachably connected to the handle element (3), the voice control module (6) includes a computer unit (14) which is electrically connected to the at least one acoustic sensor (29), **characterized in that** the computer unit (14) is configured to generate control signals for controlling the surgical light (10) depending on the measuring data determined by means of the at least one acoustic sensor (29).

2. The handle device (1) according to claim 1, **characterized in that** the voice control module (6) includes a housing (8) in which the at least one acoustic sensor (29) is received.

3. The handle device (1) according to claim 2, **characterized in that** the housing (8) includes a disk-shaped hollow portion (9) inside of which the at least one acoustic sensor (29) is arranged.

4. The handle device (1) according to claim 2 or 3, **characterized in that** the housing (8) is transmissive to an acoustic signal to be detected by the at least one acoustic sensor (29) in the area of the at least one acoustic sensor (29).

5. The handle device (1) according to one of the claims 1 to 4, **characterized in that** the voice control module (6) includes a handle receiving portion (11) which is detachably connected to the handle element (3).

6. The handle device (1) according to claim 5, **characterized in that** the handle receiving portion (11) forms a receiving pivot (12) to which a sleeve portion (13) of the handle element (3) including the handle surface (5) is attached in at least one operating state of the handle device (1).

7. The handle device (1) according to one of the claims 1 to 6, **characterized in that** the handle element (3) is connected to the voice control module (6) by form fit and/or force fit in at least one operating state.

8. The handle device (1) according to one of the claims 1 to 7, **characterized in that** in the voice control module (6), at least one distance sensor (7) designed for detecting a position of an object (25) is contained.

9. The handle device (1) according to claim 8, **characterized in that** the at least one distance sensor (7) is electronically connected to the computer unit (14).

10. The handle device (1) according to one of the claims 1 to 9, **characterized in that** the voice control module (6) is connected to an image-recording device.

11. A surgical light (10) comprising a handle device (1) according to one of the claims 1 to 10, wherein the handle device (1) is at least partially detachably connected to a receiving body (2) of the surgical light (10).

12. The surgical light (10) according to claim 11, **characterized in that** the voice control module (6) is detachably attached to the receiving body (2).

13. The surgical light (10) according to claim 11 or 12, **characterized in that** the computer unit (14) of the voice control module (6) is wire-connected to a central control unit (40) of the surgical light (10).

14. The surgical light (10) according to claim 11 or 12, **characterized in that** the computer unit (14) of the voice control module (6) is connected to a central control unit (40) of the surgical light (10) by means of wireless data communication.

## Revendications

1. Dispositif de poignée (1), qui est prévu pour être monté sur un corps récepteur de lumière (2) d'une lampe scialytique (10), doté d'un élément de préhension (3), qui est formé au niveau d'une zone extérieure (4) d'une surface de préhension (5), dans lequel
un module de commande vocale (6) présentant au moins un capteur sonore (29) est raccordé de manière amovible à l'élément de préhension (3), dans lequel le module de commande vocale (6) présente en outre une unité de calcul (14), laquelle unité de calcul (14) est raccordée électriquement à l'au moins un capteur sonore (29),
**caractérisé en ce que** l'unité de calcul (14) est formée pour produire des signaux de commande pour commander la lampe scialytique (10) en fonction des données de mesure déterminées au moyen de l'au moins un capteur sonore (29).

2. Dispositif de poignée (1) selon la revendication 1, **caractérisé en ce que** le module de commande vocale (6) présente un boîtier (8), dans lequel l'au moins un capteur sonore (29) est logé.

3. Dispositif de poignée (1) selon la revendication 2, **caractérisé en ce que** le boîtier (8) présente une section de corps creux (9) en forme de disque, à l'intérieur de laquelle l'au moins un capteur sonore (29) est disposé.

4. Dispositif de poignée (1) selon la revendication 2 ou 3, **caractérisé en ce que** le boîtier (8) est formé dans la zone de l'au moins un capteur sonore (29) de manière perméable à un signal sonore à détecter par l'au moins un capteur sonore (29).

5. Dispositif de poignée (1) selon l'une quelconque des revendications 1 à 4, **caractérisé en ce que** le module de commande vocale (6) présente une section de réception de poignée (11), qui est raccordée de manière amovible à l'élément de préhension (3).

6. Dispositif de poignée (1) selon la revendication 5, **caractérisé en ce que** la section de réception de poignée (11) forme un tourillon de réception (12), sur lequel dans au moins un état de fonctionnement du dispositif de poignée (1), une section de pochette (13) présentant la surface de préhension (5) de l'élément de préhension (3) est emboîtée.

7. Dispositif de poignée (1) selon l'une quelconque des revendications 1 à 6, **caractérisé en ce que** l'élément de préhension (3) est raccordé par complémentarité de formes et/ou de force avec le module de commande vocale (6) dans au moins un état de fonctionnement.

8. Dispositif de poignée (1) selon l'une quelconque des revendications 1 à 7, **caractérisé en ce que** dans le module de commande vocale (6) est contenu au moins un capteur de distance (7) qui est conçu pour détecter une position d'un objet (25).

9. Dispositif de poignée (1) selon la revendication 8, **caractérisé en ce que** l'au moins un capteur de distance (7) est raccordé électroniquement à l'unité de calcul (14).

10. Dispositif de poignée (1) selon l'une quelconque des revendications 1 à 9, **caractérisé en ce que** le module de commande vocale (6) est raccordé à un appareil d'enregistrement d'image.

11. Lampe scialytique (10) dotée d'un dispositif de poignée (1) selon l'une quelconque des revendications 1 à 10, dans laquelle le dispositif de poignée (1) est raccordé au moins partiellement de manière amovible à un corps récepteur de lumière (2) de la lampe scialytique (10).

12. Lampe scialytique (10) selon la revendication 11, **caractérisée en ce que** le module de commande vocale (6) est monté de manière amovible sur le corps récepteur de lumière (2).

13. Lampe scialytique (10) selon la revendication 11 ou 12, **caractérisée en ce que** l'unité de calcul (14) du module de commande vocale (6) est raccordée par un fil à une unité de commande centrale (40) de la lampe scialytique (10).

14. Lampe scialytique (10) selon la revendication 11 ou 12, **caractérisée en ce que** l'unité de calcul (14) du module de commande vocale (6) est raccordé au moyen d'une connexion de données sans fil à une unité de commande centrale (40) de la lampe scialytique (10).
